# EUROPEAN PATENT APPLICATION

(11) **EP 1 808 070 A1**
(43) Date of publication of application: **18.07.2007**
(21) Application number: 05774368.4
(22) Date of filing: 21.07.2005
(51) Int. Cl.: A01K 67/027, C12N 15/86, C12N 15/63

(54) **ANIMAL MODEL OF NEURODEGENERATIVE DISEASES, METHOD OF OBTAINING SAME AND USES THEREOF**

(30) Priority: 04.08.2004 ES 200401946
(71) Applicant: INSTITUT PASTEUR, 75724 Paris (FR)
(72) Inventor: TORRES ALEMAN, Ignacio Ins. de Neurob. Ramón y Cajal, E-28002 Madrid (ES); CARRO DÍAZ, Eva María Ins. de Neurob. Ramón y Cajal, E-28002 Madrid (ES); TREJO PÉREZ, José, L. Ins. de Neurob. Ramón y Cajal, E-28002 Madrid (ES); SPUCH CALVAR, Carlos Ins. de Neurob. Ramón y Cajal, E-28002 Madrid (ES); HEARD, Jean-Michel, Inserm CR1, 75654 Paris Cédex 13 (FR); BOHL, Delphine, CR1 Inserm, 75654 Paris Cedex 13 (FR)
(74) Representative: Ungria Lopez, Javier
(86) International application number: PCT/ES2005/070106
(87) International publication number: WO 2006/015996

(57) **Abstract**

The non human animal is described as an experimental model for neurodegenerative diseases. This animal model shows an alteration in the biological activity of the IGF-I trophic factor receptor found in the epithelial cells in the choroid plexus of the cerebral ventricles. The described animal model can be obtained through a process of transgenesis. This animal model is useful for the study of ethiopathogenic neurodegenerative diseases, among them those diseases involving dementia, such as Alzheimer, or for the identification and evaluation of therapeutic compounds to combat said diseases.

## Description

### FIELD OF THE INVENTION

The invention is related, in general, to the treatment of neurodegenerative diseases and, in particular, with the development of non-human animals useful as models of neurodegenerative diseases.

### BACKGROUND OF THE INVENTION

The development of experimental models of neurological diseases is of major importance for biomedical research (Cenci MA, Whisaw IQ, Schallert T (2002) Animal models of neurological deficits: how relevant is the rat? Nat Rev Neurosci 3: 574-579). For the case of the neurodegenerative diseases, the development of models nearing the characteristics of the disease in human beings has mean a major methodological advancement. However, on all thereof being produced by conventional genetic engineering, the economic and the personnel and facility-related resources necessary are usually quite large-scale. Although, despite this, the use thereof is becoming widespread, the high cost thereof is generating a tremendous load on the resources devoted to research.

Alzheimer's disease, a typical case of neurodegenerative disease presenting dementia, is the fourth-ranked cause of death in the industrialized countries, with around 13 million individuals affected, a number which could be even greater due to approximately 25% of cases not being diagnosed. The prognosis for the upcoming years is a spiraling rise in the number of those affected, which could exceed 40 million in the industrialized countries where the population is found to be aging (Dekosky et al. (2001) Epidemiology and Pathophysiology of Alzheimer's disease, Clinical Cornerstone 3 (4): 15-26). There are currently few medications effective for treating Alzheimer's disease, and the cost of the treatment of this disease per patient is currently quite expensive, being estimated at around US $225,000, according to data from the American Alzheimer Association. The existence of this serious health problem with a highly limited number of useful medications has prompted research aimed to ascertaining the etiopatogenic mechanism of said neurodegenerative disease for the purpose of identifying and evaluating potentially therapeutic compounds to combat this disease.
In the case of Alzheimer's disease, one of the main advancements has come precisely on being able to identify the proteins involved in the familial Alzheimer's disease, which is not associated with aging as is sporadic Alzheimer's disease, which is, by far, the most frequent form of this disease (Mayeux R (2003) Epidemiology of neurodegeneration. Annu Rev Neurosci 26: 81-104).

Transgenic models which are carriers of the different mutations found in familial Alzheimer's disease patients, such as presenilins and amyloid beta (Hock BJ, Jr., Lamb BT (2001) transgenic mouse models of Alzheimer's disease. Trends Genet 17: S7-12). One highly important drawback is that although these mutant animals have several symptoms of Alzheimer's disease, none of them shows the full spectrum of pathological changes associated with this disease (Richardson JA, Burns DK (2002) Mouse models of Alzheimer's disease: a quest for plaques and tangles. ILAR J 43: 89-99). In an attempt to solve this problem, transgenic mouse strains with the different mutations which each recreate different aspects of the disease have been crossed with one another in order to thus achieve a model which better resembles the human pathology (Phinney AL, Home P, Yang J, Janus C, Bergeron C, Westaway D (2003) Mouse models of Alzheimer's disease: the long and filamentous road. Neurol Res 25: 590-600). For example, crossing mice which express major amounts of one of the mutated forms of the precursor protein of human amyloid beta (APP-Swe695) with mice which express mutated forms of presenilins generate hybrids which has amyloid plaques along with neurofibrillary tangles and cognitive deficits (Duff K, Eckman C, Zehr C, Yu X, Prada CM, Perez-tur J, Hutton M, Buee L, Harigaya Y, Yager D, Morgan D, Gordon MN, Holcomb L, Refolo L, Zenk B, Hardy J, Younkin S (1996) Increased amyloid-beta42(43) in brains of mice expressing mutant presenilin 1. Nature 383: 710-713; Richards JG, Higgins GA, Ouagazzal AM, Ozmen L, Kew JN, Bohrmann B, Malherbe P, Brockhaus M, Loetscher H, Czech C, Huber G, Bluethmann H, Jacobsen H, Kemp JA (2003) PS2APP Transgenic Mice, Coexpressing hPS2mut and hAPPswe, Show Age-Related Cognitive Deficits Associated with Discrete Brain Amyloid Deposition and Inflammation. J Neurosci 23: 8989-9003).

In following, an indication is provided, for illustrative purposes, of some of the patents related to animal models of Alzheimer's disease : US20030229907. Transgenic non-human mammals with progressive neurologic disease; US20030167486, Double transgenic mice overexpressing human beta secretase and human APP-London; US20030145343, Transgenic animals expressing human p25; US20030131364, Method for producing transgenic animal models with modulated phenotype and animals produced therefrom; US20030101467, Transgenic animal model for Alzheimer disease; US200030093822, Transgenic animal model of neurodegenerative disorders; US6,717,031, Method for selecting a transgenic mouse model of Alzheimer's disease; US6,593,512, Transgenic mouse expressing human tau gene; US6,563,015, Transgenic mice over-expressing receptor for advanced alycation endproduct (RAGE) and mutant APP in brain and uses thereof; US6,509,515, Transgenic mice expressing mutant human APP and forming congo red staining plaques; US6,455,757, Transgenic mice expressing human APP and TGF-beta demonstrate cerebrovascular amyloid deposits; US6,452,065, Transgenic mouse expressing non-native wild-type and familial Alzheimer's Disease mutant presenilin 1 protein on native presenilin 1 null background; WO03053136, Triple transgenic model of Alzheimer disease; WO03046172, Disease model; US6563015, Transgenic mice over-expressing receptor for advanced glycation endproduct (RAGE) and mutant APP in brain and uses thereof; WO0120977, Novel animal model of Alzheimer disease with amyloid plaques and mitochondrial dysfunctions; EP1285578. Transgenic animal model of Alzheimer's disease.

At present, these transgenic animal models are the only ones accepted for the study of pathogenic mechanisms of Alzheimer's disease and for the screening, at the pharmaceutical level, of new drugs. Given the disparity of models which have been generated for the purpose of recreating and analyzing each one of the possible causes of this disease, the availability thereof is restricted in many cases due to property right-related questions and, above all, due to the lack of material resources necessary for generating complex hybrids (Oddo S, Caccamo A, Shepherd JD, Murphy MP, Golde TE, Kayed R, Metherate R, Mattson MP, Akbari Y, LaFerla FM (2003) Triple-transgenic model of Alzheimer's disease with plaques and tangles: intracellular Aβ and synaptic dysfunction. Neuron 39: 409-421). This means severe limitations on the widespread use of these models.
Additionally worthy of special mention is the fact that the mediations currently existing for the treatment of Alzheimer's disease are not very effective and that the models based on existing transgenic animals have deficiencies on not being a true reflection of the pathology of Alzheimer's disease. Therefore, a serious health problem continues to exist with a highly limited number of useful medications, the need therefore existing of developing experimental models alternative to the existing ones which afford the possibility of studying the etiopathogenic mechanism of said neurodegenerative disease and/or of identifying and evaluating potentially therapeutic compounds to combat said disease.

On the other hand, the growth factor receptor similar to Type I insulin (IGF-1) is a membrane protein pertaining to the family of receptors with tyrosin-kinase enzymatic activity, quite similar to the insulin receptor (Ullrich A, Gray A, Tam AW, Yang-Feng T, Tsubokawa M, Collins C, Henzel W, Le Bon T, Kathuria S, Chen E. (1986) Insulin-like growth factor I receptor primary structure: comparison with insulin receptor suggests structural determinants that define functional specificity. EMBO J5: 2503-2512). The ample and highly relevant biological functional have led to its being studied intensively such that the intracellular signaling pathway is relatively well-known (LeRoith D, Werner H, Beitner-Johnson D, Roberts CT, Jr. (1995) Molecular and cellular aspects of the insulin-like growth factor I receptor. Endocr Rev 16:143-163). The role thereof in pathologies such as cancer, diabetes and neurodegeneration were on target in the search for pharmacological modulators of clinical use, although the etiopathogenic role is not know, in pathologies such as Alzheimer's disease, which the functional alteration thereof may induce.

### SUMMARY OF THE INVENTION

The invention confronts the problem of providing new animal models of human neurodegenerative diseases, such as human neurodegenerative diseases which present dementia, one of which is Alzheimer's disease.

The solution provided by this invention is based on the inventors having observed that the repression of the functional activity of the IGF-1 receptor in the epithelial cells of the choroid plexa of the ventricles of an animal's brain makes the development of an animal model of neurodegenerative diseases possible, in general and in particular, an animal model of human neurodegenerative diseases which present with dementia, such as Alzheimer's disease, which fulfills the main characteristics of said human disease, which is simple to produce and which can be used in laboratory animals with any genetic background. For this purpose, and among other technical possibilities, a vector containing a mutated form of the IGF-1 receptor which nullifies the functional activity of this trophic factor at the level of the choroid plexus on serving as a negative dominant (Example 1) was injected by means of stereotaxic surgery into the lateral ventricles of the brain. A few months later, the animal showed all of the symptoms associated with Alzheimer's disease: accumulation of amyloid peptide in the brain, hyperphosphorylated tau protein deposits in conjunction with ubiquitin, loss of synaptic proteins and severe cognitive deficits (learning and memory). The development of the Alzheimer-type pathology appears 3-6 months following the injection of the vector, depending upon the genetic background of the host animal, such that in the genetically-engineered animals which can potentially modulate the onset of Alzheimer's disease, the standard neuropathology of said disease appears earlier (Examples 2 and 3).

Therefore, in one aspect, the invention is related to a non-human animal useful as an experimental model characterized in that it shows an alteration in the biological activity of the IGF-1 receptor located in the epithelial cells of the choroid plexus of the cerebral ventricles. Said non-human animal is useful as an experimental model of neurodegenerative diseases, particularly human neurodegenerative disease which present with dementia, such as Alzheimer's Disease.

In another aspect, the invention is related to a procedure for the production of said non-human animal useful as an experimental model which includes the repression of the functional activity of the IGF-1 receptor in the epithelial cells of the choroid plexus of said non-human animal by means of a transgenesis process. For this purpose, it is necessary for gene structures and vectors to be developed, which, in conjunction with the applications thereof, constitute additional aspects of the present invention.
In another aspect, the invention is related to the use of said non-human animal as an experimental model for the study of the etiopathogenic mechanism of a neurodegenerative disease or for the identification and evaluation of therapeutic compounds to combat said disease. In one particular embodiment, said neurodegenerative disease is a human neurodegenerative disease which presents with dementia, such as Alzheimer's disease.

One of the advantages of the experimental model developed by this invention lies in that it is a perfectly true reflection of the pathology of Alzheimer's disease, as a result of which said model is a qualitative leap forward in the study of the etiopathogenic mechanism of said neurodegenerative disease as well as in the development of effective tools for the identification and evaluation of therapeutic compounds to combat said disease.

### BRIEF DESCRIPTION OF THE FIGURES

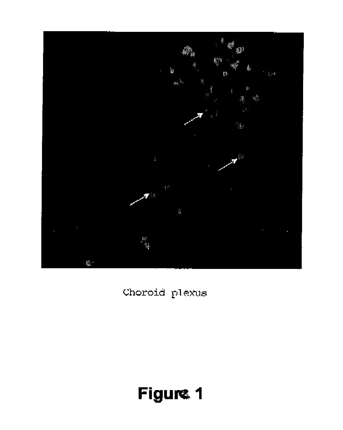

Figure 1 is a photo showing that the HIV/GFP lentiviral vector allows the expression of the transgene in the choroid plexus cells, the expression of green fluorescent protein GFP (green) being seen in cells of the choroid plexus (arrows) of adult rat following intracerebroventricular (icv) injection of the HIV/GFP vector. The photo shows sells of the choroid plexus of an animal which was administered, three months prior to be sacrificed, one single icv injection of the HIV/GFP lentiviral vector.

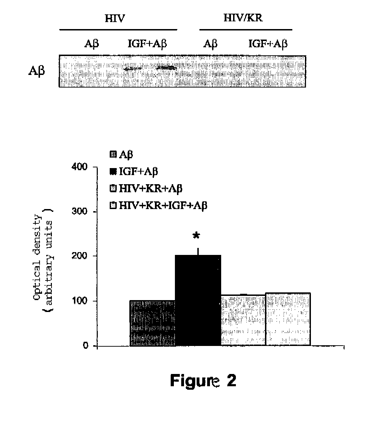

Figure 2 shows that the administration of the HIV/IGF-IR.KR (HIV/KR) vector to epithelial cells in culture taken from the choroid plexus of postnatal rats generates a loss of response to the IGF-1. Only in cells infected with KR (HIV+KR+ and HIV+KR+IGF+Aβ), but not in those transfected with a null HIV vector (HIV) the IGF-1 does not promote transcytosis of peptide Ap-40. *P<0.05 vs. all of the other groups.

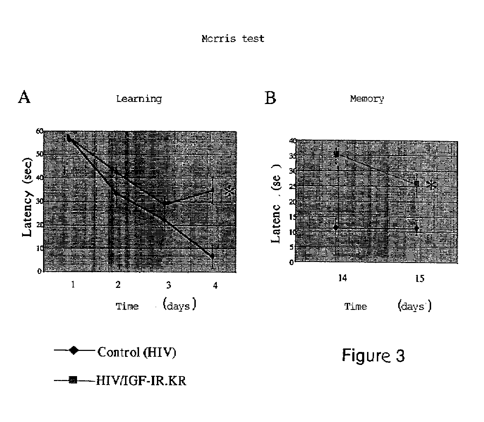

Figure 3 shows that the learning (A) and the spatial memorization (B) are decreased in HIV/IGF-IR.KR rats, given that the latter learn more slowly and worse than the control rats (HIV) in the Morris test, consisting of memorizing the position of a platform covered with water in a pool where the animal swims without being able to rest anywhere else but on the platform. HIV/IGF-IR.KR rats: r²=0.8516 vs. control rats r²=0.9884, *P<0,05.

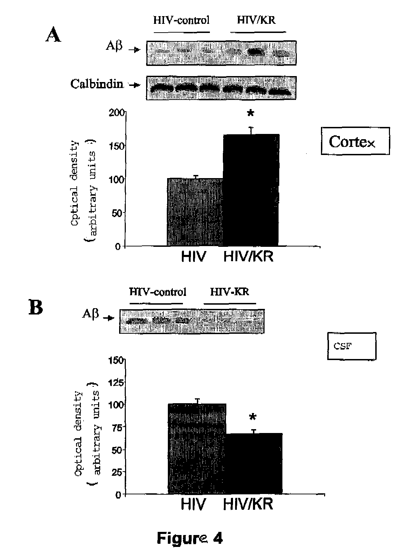

Figure 4 shows the Aβ levels in cerebral cortex (A) and in cerebrospinal fluid (CSF) (B) of rats injected with the HIV/IGF-IR-KR (HIV/KR) vector. Whilst an increase in produced in the cerebral levels of Aβ, there is a parallel decrease in the CSF, indicating a decrease in the Aβ clearance. The levels were determined by immunoblot densitometry using anti- Aβ antibodies. Representative immunoblots are shown. Levels of calbindin, a neuronal protein, are also evaluated to show the differences are not due to the amount of total protein in each experimental group. *P<0.05 vs, control (rats injected with null HIV).

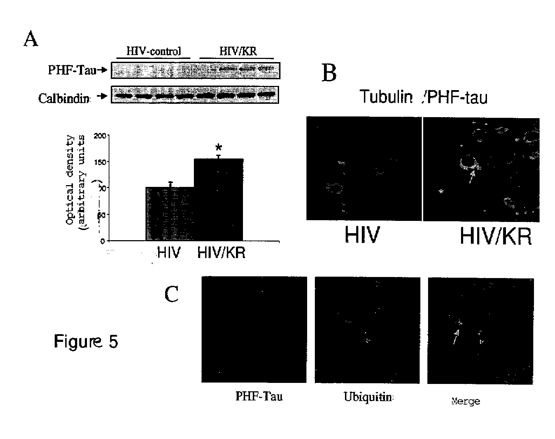

Figure 5 shows the levels of hyperphosphorolyated tau (HPF-tau) in the cerebral cortex of rats injected with the HIV71GF-IR-KR (HIV/KR) vector. Figure 5° shows the levels of HPF-tau in the cerebral cortex of rats injected with the HIV/IGF-IR.KR (HIW7KR) vector and with the control vector (HIV-control)- The levels were determined by immunoblot densitometry with anti-HPF-tau antibodies. *P<0.05 vs. control. Figure 5B shows the results of a confocal microscopy analysis of the tissular location of the HPT-tau deposits. The HIV/IGF-IR-.KR (HIV/KR) animals (right panel), but not the control animals (treated with HIV, left panel), show accumulations of HPF-tau (red) both inside (arrow) and outside (asterisk) of the neurons (immunopositive for beta-tubulin, in green) in areas of the telencephalon. The yellow-red intracellular signal is revealing of the colocalization of HPF-tau in neurons. Figure 5C shows that the extracellular accumulations of HPF-tau also contain ubiquitin. A colocalization (yellow accumulations, arrow) of HPF-tau deposits (red) with ubiquitin (green) is produced. The control animals do not have these deposits (data not shown).

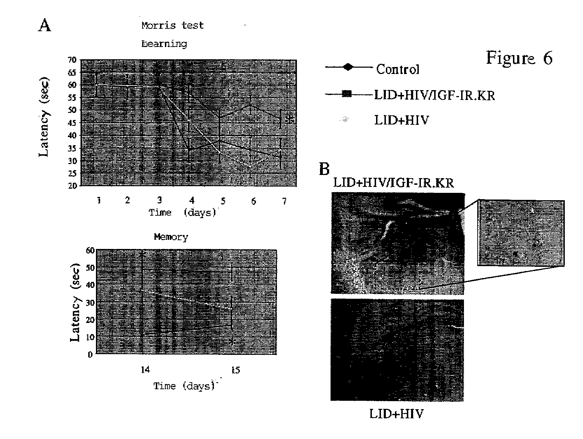

Figure 6 shows a standard Alzheimer neuropathology in mice with modified genetic background. Figure 6 A shows that the old (over 15 months) LID mice treated with the HIV/IGF-IR.KR (HIV/KR) [LID-HIV/IGF-IR.KR) vector practically did not learn the Morris test. Whilst the old or LID mice which were administered only the control viral vector [LID-HIV] learned and retained what they had learned. Similarly, the LID-HIV/IGF-IR.KR mice, where the signaling of the IGF-1 receptor in the choroid plexus has been eliminated, learn significantly worse (*P<0.001 vs. controls). LID-HIV/IGF-IR.KR (n=5): r²=0.6320, LID-HIV (n=7): r²=0.7379; Controls of the same age (n=6), r²=0.7909. Figure 6B shows that the LID-HIV/IGF-IR.KR animals show accumulations of Aβ, marked with asterisks on the zoom panel) in telecephalon areas which are barely found in the LID-HIV control mice (lower panel).

### DETAILED DESCRIPTION OF THE INVENTION

One aspect of the present invention is related to a non-human animal useful as an experimental model, referred to hereinafter as animal model of the invention, characterized in that it has an alteration in the biological activity of the growth factor receptor similar to Type I insulin (IGF-1) located in the epithelial cells of the choroid plexus of the cerebral ventricles.

As used in the present invention, the term "non-human animal" refers to a non-human mammal of any genetic background, preferably laboratory animals such as rodents, more preferably rats and mice or non-human primates.

As used in the present invention, the term "any genetic background" refers both to a normal non-human animal and to a transgenic non-human animal.

The term "normal", applied to animal, as used in the present invention, refers to animals having no transgenes which could be involved in the etiopathogenia of neurodegenerative diseases, for example, human neurodegenerative diseases, for example, human neurodegenerative disease which present with dementia, such as Alzheimer's disease.
The term "transgenic", applied to animal, as used in the present invention, refers to animals which contain a transgene which could be involved in the etiopathogenia of neurodegenerative diseases, for example, human neurodegenerative diseases which present with dementia, such as Alzheimer's disease, and includes, for illustrative purposes without limiting the scope of the present invention, transgenic animals of the following group: LID mice (Yakar S, Liu JL, Stannard B, Butler A, Accili D, Sauer B, LeRoith D (1999) Normal growth and development in the absence of hepatic insulin-like growth factor I, Proc Natl Acad Sci USA 96: 7324-7329) transgenic animals carriers of mutations in presenilins and beta amyloid (Hock BJ, Jr., Lamb BT (2001) Transgenic mouse models of Alzheimer's disease. Trends Genet 17: S7-12), animals carriers of other mutations and alterations (US20030229907, Transgenic non-human mammals with progressive neurologic disease; US20030145343, Transgenic animals expressing human p25; US20030131364, Method for producing transgenic animal models with modulated phenotype and animals produced therefrom; US20030101467, Transgenic animal model for Alzheimer disease; US200030093822, Transgenic animal model of neurodegenerative disorders; US6,717,031, Method for selecting a transgenic mouse model of Alzheimer's disease: US6,593,512. Transgenic mouse expressing human tau gene; US6,563,015, Transgenic mice over-expressing receptor for advanced alycation endproduct (RAGE) and mutant APP in brain and uses thereof; US6,509,515, Transgenic mice expressing mutant human APP and forming congo red staining plaques; US6,455,757, Transgenic mice expressing human APP and TGF-beta demonstrate cerebrovascular amyloid deposits; US6,452,065, Transgenic mouse expressing non-native wild-type and familial Alzheimer's Disease mutant presenilin 1 protein on native presenilin 1 null background; WO03053136, Triple transgenic model of Alzheimer disease; WO03046172, Disease model; US6563015, Transgenic mice over-expressing receptor for advanced glycation endproduct (RAGE) and mutant APP in brain and uses thereof; WO0120977, Novel animal model of Alzheimer disease with amyloid plaques and mitochondrial dysfunctions; EP1285578, Transgenic animal model of Alzheimer's disease) and transgenic animals produced by way of the crossing of strains of transgenic mice with the different mutations which take place in Alzheimer's disease (Phinney AL, Home P, Yang J, Janus C, Bergeron C, Westaway D (2003) Mouse models of Alzheimer's disease: the long and filamentous road. Neurol Res 25: 590-600; Duff K, Eckman C, Zehr C, Yu X, Prada CM, Perez-tur J, Hutton M, Buee L, Harigaya Y, Yager D, Morgan D, Gordon MN, Holcomb L, Refolo L, Zenk B, Hardy J, Younkin S (1996) Increased amyloid-beta42(43) in brains of mice expressing mutant presenilin 1. Nature 383: 710-713; Richards JG, Higgins GA, Ouagazzal AM, Ozmen L, Kew JN, Bohrmann B, Malherbe P, Brockhaus M, Loetscher H, Czech C, Huber G, Bluethmann H, Jacobsen H, Kemp JA (2003) PS2APP Transgenic Mice, Coexpressing hPS2mut and hAPPswe, Show Age-Related Cognitive Deficits Associated with Discrete Brain Amyloid Deposition and Inflammation. J Neurosci 23: 8989-900; US20030167486 Double transgenic mice overexpressing human beta secretase and human APP-London).

The alteration of the biological activity of the IGF-I receptor function in the epithelial cells of the choroid plexus of the cerebral ventricle of the animal model of the invention will consist, in general, of the functional repression of the biological activity thereof (biological repression).
Said alteration of the biological activity of the IGF-1 receptor function in the epithelial cells of the choroid plexus of the cerebral ventricles may be due to a repression of the functional activity of the IGF-I receptor due to the expression of a polynucleotide the sequence of nucelotides of which encodes a dominant non-functional mutated form of the IGF-I receptor. In one particular embodiment, said polynucleotide encodes a dominant non-functional mutated form of the human IGF-I receptor. For illustrative purposes, said dominant non-functional mutated form of the human IGF-1 receptor is selected between the nonfunctional mutated form of the IGF-1 receptor referred to as IGF-IR.KR, which has the K1003R mutation, in which the lysine residue of position 1003 of the amino acid sequence of the human IGF-I receptor has been substituted for an arginine residue and the nonfunctional mutated form of the IGF-I receptor referred to as IGF-IR.KA, which has the K1003A mutation, in which the lysine residue in position 1003 of the amino acid sequence of the human IGF-I receptor has been substituted for an alanine residue (Kato H, Faria TN, Stannard B, Roberts CT, Jr., LeRoith D (1993) Role of tyrosine kinase activity in signal transudation by the insulin-like growth factor-I (IGF-I) receptor. Characterization of kinase-deficient IGF-I receptors and the action of an IGF-I-mimetic antibody (alpha IR-3). J Biol Chem 268: 2655-2661). The numbering system used for numbering the amino acid residues of the human IGF-I receptor is that used by Ullrich et al. (Ullrich A. et al. 1985) Human insulin receptor and its relationship to the tyrosine kinase family of oncogenes. Nature 313:756-761; Ullrich A. et al. (1986) Insulin-like growth factor I receptor primary structure: comparison with insulin receptor suggests structural determinants that define functional specificity. EMBO J. 1986 Oct; 5(10): 2503-2512).

Alternatively, said alteration of the biological activity of the IGF-I receptor function in the epithelial cells of the choroid plexus of the cerebral ventricles can be due to the repression of the functional activity of the IGF-I receptor due to the expression of a polynucleotide the sequence of nucleotides of which encodes an element inhibiting the expression of the gene of the IGF-I receptor capable of repressing the functional activity thereof. As used in the present invention, the term "element inhibiting the expression of the IGF-I receptor gene capable of repressing the functional activity thereof' refers to a protein, enzymatic activity or sequence of nucleotides, RNA or DNA, single or double-strand, which inhibits the translation into protein of the mRNA of the IGF-I receptor. For illustrative purposes, said polynucleotide can be a polynucleotide which encodes a specific sequence of antisense nucleotides of the sequence of the gene or of the mRNA of the IGF-I receptor, or rather a polynucleotide which encodes a specific aptamer of the mRNA of the IGF-I receptor, or rather a polynucleotide which encodes a specific interference RNA ("small interference RNA" or siRNA) of the mRNA of the IGF-I receptor.

The animal model of the invention can have any genetic background; nevertheless, in one particular embodiment, said animal model of the invention comes from a normal animal, advantageously, from a healthy normal animal, in other words, which has no diagnosed pathology, such as a healthy rat (Example 2), whilst in another particular embodiment of the invention, it comes from a transgenic animal, such as an LID transgenic mouse (Example 3).

The animal model of the invention is an animal useful as an experimental model of neurodegenerative diseases, for example, neurodegenerative diseases which present with dementia. Preferably, said neurodegenerative diseases are human neurodegenerative diseases, more preferably human neurodegenerative diseases which present with dementia. In one particular embodiment, said human neurodegenerative disease which presents with dementia is Alzheimer's disease. Alzheimer's disease totals 60% of the dementia cases, whilst microvascular or multi-infarct disease totals 20% thereof. Other minor causes of dementia are chronic alcohol and drug abuse and very low-incidence neurological disease, such as Pick's disease and Creutzfeldt-Jacob disease.

Therefore, in another aspect, the invention is related to the use of the animal model of the invention as an experimental model of neurodegenerative diseases, such as neurodegenerative diseases which present with dementia; preferably, said neurodegenerative diseases are human neurodegenerative diseases, such as human neurodegenerative diseases which present with dementia; for example, Alzheimer's disease.

Likewise, the use of the animal model of the invention for the study of the etiopathogenic mechanisms of neurodegenerative diseases, particularly human neurodegenerative diseases and, more particularly, human neurodegenerative diseases which present with dementia, such as Alzheimer's disease, as well as the use of the animal model of the invention for the identification and evaluation of potentially therapeutic compounds to combat said diseases constituting additional aspect of the present invention.
The animal model of the invention can be produced by means of a transgenesis process allows the functional repression of the IGF-I receptor in the epithelial cells of the choroid plexus of said animal model of the invention.

Therefore, in another aspect, the invention is related to a procedure for the production of the animal model of the invention, referred to hereinafter as the procedure of the invention, which includes the repression of the functional activity of the IGF-I receptor of the epithelial cells of the choroid plexus of said animal model of the invention by means of a transgenesis process.

As used in the present invention, the term "transgenesis process" refers to any technique or procedure which permits the integration of an exogenous gene or "transgene" into a series of cells of a live organism without affecting al of the cells of said organism, and which confers a new biological property upon said cells and upon the organism carrying the same. Said transgene or exogenous gene refers to a DNA normally not resident or present in the cell which is aimed at being transformed.

On the other hand, the transgenesis process for producing the animal model of the invention can be applied both to fully-developed animals and to embryos thereof provided that it permit the repression of the functional activity of the IGF-I receptor in the epithelial cells of the choroid plexus of said fully-developed animal model.

In one particular embodiment, said transgenesis process which leads to the repression of the functional activity of the IGF-I receptor includes the transformation of epithelial cells of the choroid plexus of a fully-developed non-human animal such that they express a dominant non-functional mutated form of the IGF-I receptor. This objective can be achieved by means of the administration to epithelial cells of the choroid plexus of said non-human animal of a gene structure which includes a polynucleotide the nucleotide sequence of which encodes a dominant non-functional mutated form of the IGF-I receptor for the purpose of transforming said epithelial cells of the choroid plexus so that they will express said dominant non-functional mutated form of the IGF-I receptor. Advantageous, said gene structure is included within a vector, such as, for example, an expression vector or a transference vector.

As used in the present invention, the term "vector" refers to systems utilized in the transference process of an exogenous gene or of an exogenous gene structure to the inside of a cell, thus permitting the stable vehiculation of genes and exogenous gene structures. Said vectors can be non-viral vectors or viral vectors, preferably viral vectors given that the transgenesis with viral vectors has the advantage of being able to direct the expression of a foreign gene in adult tissues relatively precisely and is one of the reasons why the general use thereof for gene therapy is being posed ( Pfeifer A, Verma IM (2001) Gene therapy: promises and problems. Annu Rev Genomics Hum Genet 2: 177-211).

The invention has been exemplified by means of the use of lentiviral vectors. These vectors are easy to handle, one of the main advantages thereof being their effective transduction, their genomic integration and their persistent or prolonged expression. Other appropriate viral vectors include retroviral, adenoviral or adenoassociated vectors (Consiglio A, Quattrini A, Martino S, Bensadoun JC, Dolcetta D, Trojani A, Benaglia G, Marchesini S, Cestari V, Oliverio A, Bordignon C, Naldini L (2001) In vivo gene therapy of metachromatic leukodystrophy by lentiviral vectors: correction of neuropathology and protection against learning impairments in affected mice. Nat Med 7: 310-316; Kordower JH, Emborg ME, Bloch J, Ma SY, Chu Y, Leventhal L, McBride J, Chen EY, Palfi S, Roitberg BZ, Brown WD, Holden JE, Pyzalski R, Taylor MD, Carvey P, Ling Z, Trono D, Hantraye P, Deglon N, Aebischer P (2000) Neurodegeneration prevented by lentiviral vector delivery of GDNF in primate models of Parkinson's disease. Science 290: 767-773). Examples of lentiviral vectors include the type 1 human immunodeficiency virus (HIV-1), of which numerous appropriate vectors have been developed. Other lentiviruses appropriate for their use as vectors include the primate lentivirus group including the type 2 human immunodeficiency virus (HIV-2), the 3 human immunodeficiency virus (HIV-3), the simian immunodeficiency virus (SIV), the simian AIDS retrovirus (SRV-1), the type 4 human T-cell lymphotrophic virus (HTLV4), as well as the bovine lentivirus, equine lentivirus, feline lentivirus, ovine/caprine lentivirus and murine lentivirus groups.

The invention provides a vector, such as a viral vector, specifically a lentiviral vector, useful for producing an animal model of the invention, which is useful as an experimental model of neurodegenerative disease, specifically, as a model of human neurodegenerative diseases which present with dementia, such as Alzheimer's disease. Said vector as well as the production thereof shall be described in greater detail at a further point herein.

The administration of said gene structure which includes a polynucleotide the nucleotide sequence of which encodes a dominant non-functional mutated form of the IGF-I receptor or of said vector which includes said gene structure, to the epithelial cells of the choroid plexus of the non-human animal to be transformed, can be carried out by many conventional method: nevertheless, in one particular embodiment, the administration of said vector to said epithelial cells of the choroid plexus is carried out by means of intracerebroventricular (icv) injection.

As used in the present invention, the term "a dominant non-functional mutated form of the IGF-I receptor" includes any mutated form of the IGF-I receptor which acts as negative dominant by recombination with the endogenous normal IGF-I receptor, repressing the biological function thereof, in the course of the procedure developed b the present invention. Said dominant non-functional mutated form of the IGF-I receptor is expressed by epithelial cells of the choroid plexus of the animal model of the invention as a result of the transformation thereof with a gene structure which includes a polynucleotide the nucleotide sequence of which encodes said dominant non-functional mutated form of the IGF-I receptor. In one particular embodiment, said polynucleotide encodes a dominant non-functional mutated form of the human IGF-I receptor. In other particular embodiments, said polynucleotide encodes a dominant non-functional mutated form of the IGF-I receptor of an animal species other than human, such as a mammal, for example a rodent or a non-human primate.

Although practically any dominant non-functional mutated form of the IGF-I receptor can be used for the purpose of achieving functional repression of the biological activity (biological repression) of the IGF-I receptor, in one particular embodiment, said dominant non-functional mutated form of the IGF-I receptor is selected among the non-functional mutated forms of the human IGF-I receptor known as IGF-IR.KR and IGF-IR.KA in this description, defined previously.

The non-human animal whose epithelial cells of the choroid plexus of the cerebral ventricles are going to be transformed by means of the administration of the transgene can have any genetic background.

The procedure of the invention is materialized, in one specific embodiment, in a procedure for the production of an animal model of the invention in which the vector utilized is the lentiviral vector of HIV-1 origin known as HIV/IGF-IR.KR (HIV/KR) in this description, the dominant non-functional mutated form of the IFG-I receptor is the nonfunctional mutated form of the human IGF-I receptor known as IGF-IR.KR, and the non-human animal whose choroid plexus epithelial cells have been transformed is a healthy adult normal rat (Example 2).

Additionally, the procedure of the invention is materialized, in another specific embodiment, in a procedure for the production of an animal model of the invention in which the vector utilized is the lentiviral vector of known as HIV/IGF-IR.KR (HIV/KR), the dominant non-functional mutated form of the IFG-I receptor is the nonfunctional mutated form of the human IGF-I receptor known as IGF-IR.KR, and the non-human animal whose choroid plexus epithelial cells have been transformed is a LID transgenic mouse (Example 3).

Alternatively, as previously mentioned hereinabove, the alteration of the biological activity of the function of the IGF-I receptor in the epithelial cells of the choroid plexus of the cerebral ventricles can be due to the repression of the functional activity of the IGF-I receptor due to the expression of a polynucleotide the nucleotide sequence of which encodes an element inhibiting the expression of the IGF-I receptor gene capable of repressing the functional activity thereof.

Therefore, in another particular embodiment, said transgenesis process of repressing the functional activity of the IGF-I receptor includes the transformation of epithelial cells of the choroid plexus of a non-human animal by means of the introduction of a gene structure which includes a polynucleotide the nucleotide sequence of which encodes an element inhibiting the expression of the gene of the IGF-I receptor capable of repressing the biological activity thereof, said inhibiting element being selected among:
a) a specific antisense nucleotide sequence of the gene sequence or of the mRNA of the IGF-1 receptors
b) a specific ribosome of the mRNA of the IGF-1 receptor
c) a specific aptamer of the mRNA of the IGF-I receptor, or
d) a specific interference RNA 8iRNA) of the mRNA of the IGF-I receptor.

Advantageous, said gene structure is included within a vector, such as, for example, an expression vector or a transference vector. The characteristics of said vector have been previously defined.

The aforementioned a)-d) nucleotide sequences prevent the expression of the gene in mRNA or of the mRNA in the protein of the IGF-1 receptor and therefore repress the biological function thereof and can be developed by an expert in the genetic engineering sector in terms of the existing know-how in the state of the art on transgenesis and gene expression repression (Clarke, A.R. (2002) Transgenesis Techniques. Principles and Protocols, 2nd Ed Humana Press, Cardiff University; Patent US20020128220. Gleave, Martin. TRPM-2 antisense therapy; Puerta, Ferández E et al. (2003) Ribozymes: recent advances in the development of RNA tools. FEMS Microbiology Reviews 27: 75-97; Kikuchi, et al., 2003. RNA aptamers targeted to domain II of Hepatitis C virus IRES that bind to its apical loop region. J. Biochem 133, 263-270; Reynolds A. et al., 2004. Rational siRNA design for RNA interference. Nature Biotechnology 22 (3): 326-330).

In another aspect, the invention is related to a vector useful for putting the procedure for producing the animal model of the invention into practice. Said vector can be a non-viral vector or, advantageously, a viral vector, as has been previously mentioned hereinabove, and includes a polynucleotide the nucleotide sequence of which encodes a dominant non-functional mutated form of the IGF-I receptor or rather a polynucleotide the nucleotide sequence of which encodes an element inhibiting the expression of the IGF-receptor gene capable of repressing the functional activity thereof, in conjunction, optionally, with the necessary elements for permitting the expression thereof in cells of non-human animals. Said vectors can be in the form of artificial or chimeric viral particles.
In one particular embodiment, said vector is a lentiviral vector which includes a polynucleotide the nucleotide sequence of which is selected between a sequence of nucleotides which encodes a dominant non-functional mutated form of the IGF-I receptor and a sequence of nucleotides which encodes an element inhibiting the expression of the IGF-I receptor gene capable of repressing the functional activity thereof.

In one particular embodiment, the sequence of nucleotides which encodes a dominant non-functional mutated form of the IGF-I receptor is selected between the nonfunctional mutated forms of the human IGF-I receptor known as IGF-IR.KR and IGF-IR.KA in this description, previously defined.

In another particular embodiment, the sequence of nucleotides which encodes an element inhibiting the expression of the IGT-I receptor gene capable of repressing the functional activity thereof is selected between a sequence which encodes. A) a specific antisense sequence of nucleotides of the gene sequence or of the mRNA of the IGF-I receptor; b) a specific ribosome of the mRNA of the IGF-I receptor; c) a specific aptamer of the mRNA of the IGF-I receptor; and d) a specific interference RNA (iRNA) of the mRNA of the IGF-I receptor.

The invention provides, in one specific embodiment, a lentiviral vector which can be obtained by means of transitory transfection in packaging cells of:
a plasmid (i) which includes a sequence of nucleotides selected between:
   - a sequence of nucleotides which encodes a dominant non-functional mutated form of the IGF-I receptor, and
   - a sequence of nucleotides which encodes an element inhibiting the expression of the gene of the IGF-I receptor capable of repressing the functional activity thereof;
a plasmid (ii) which includes the sequence of nucleotides which encodes the Rev protein;
a plasmid (iii) which includes the sequence of nucleotides which encodes the Rev response element (RRE); and
a plasmid (iv) which includes the sequence of nucleotides which encodes the heterologous packaging of the vector.

Although practically any appropriate packaging cell can be used, in one particular embodiment, said packaging cells pertain to the 293T-cell line, a line of commercially available transformed human kidney epithelial cells.

Plasmid (i) is a vector, such as a transference or expression vector, which has a gene structure which includes the transgene in question and a functional promoter in the packaging cells which make it possible for the vector being transcripted to be efficiently generated in the packaging cells. In one particular embodiment, said plasmid (i) includes a sequence of nucleotides which encodes a dominant non-functional mutated form of the IGF-I receptor selected between the non-functional mutated forms of the human IGF-I receptor referred to as IGF-IR.KR and IGF-IR.KA in this description, previously defined. In another particular embodiment, said plasmid (i) includes a sequence of nucleotides which encodes an element inhibiting the expression of the IGF-I receptor gene capable of repressing the functional activity thereof selected between a sequence which encodes: a) a specific sequence of antisense nucleotides of the sequence of the gene or of the mRNA of the IGF-I receptor; b) a specific ribosome of the mRNA of the IGF-I receptor; c) a specific aptamer of the mRNA of the IGF-I receptor; and d) a specific interference RNA (iRNA) of the mRNA of the IGF-I receptor.

Plasmid (ii) is a non-overlapping vector which virtually can contain the sequence of nucleotides which encodes any Rev protein, which promotes the cytoplasmic accumulation of the viral transcribes; nevertheless, in one particular embodiment, said plasmid (ii) is a plasmid identified as RSV-Rev, which includes the sequence of nucleotides which encodes the Rev protein of the Rous sarcoma virus (RSV).

Plasmid (iii) is a condition packaging vector and contains the sequence of nucleotides which encodes any appropriate Rev response element (RRE), to which it is joined such that the gene is expressed and the new viral particles are produced.

Plasmid (iv) contained the sequence of nucleotides which encodes the heterologous vector packaging, as a result of which it can contain the sequence of nucleotides which encodes any protein of the packaging of an appropriate virus, with the condition that said virus not be a lentivirus; nevertheless, in one particular embodiment, said plasmid is that known as p-VSV, which includes the sequence of nucleotides which encodes the packaging of the vesicular stomatitis virus (VSV).

Said lentiviral vector can be produced by conventional methods known by experts on the subject.

In one particular embodiment, said lentiviral vector is referred to as HIV7IGF-IR.KR (HIV/KR) (Example i) which allows the expression of the non-functional mutated form of the IGF-I receptor referred to as IGF-IR-KR which has a K1003R mutation in the amino acid sequence of the human IGF-I receptor, in non-human animal cells and the biological repression of the IGF-I receptor and the development of a non-human animal useful as an experimental model of human neurodegenerative diseases which present with dementia, such as Alzheimer's disease.

In another particular embodiment, said transgenesis process which leads to the repression of the functional activity of the IGF-I receptor in the epithelial cells of the choroid plexus of the animal model of the invention includes a conventional transgenesis process in the embryonic stage of said animal such that the future cells of the choroid plexus of said animal are genetically transformed and lose the capacity to respond to the IGF-I. The development of this type of transgenic animal can be carried out by an expert in the genetic engineering sector in terms of the existing know-how in the state of the art regarding transgenic animals ( Bedell MA, Jenkins NA, Copeland NG. Mouse models of human disease. Part I: techniques and resources for genetic analysis in mice. Genes Dev. 1997 Jan 1; 11(1):1-10. Bedell MA, Largaespada DA, Jenkins NA, Copeland NG. Mouse models of human disease. Part II: recent progress and future directions. Genes Dev. 1997 Jan 1; 11(1): 11-43).

One possibility of the present invention is a conventional transgenesis procedure by which the expression of a transgene which includes a specific tissue promoter (such as, for example, a transthyretin promoter, Ttr¹ (Schreiber, G. The evolution of transthyretin synthesis in the choroid plexus. Clin. Chem Lab Med. 40, 1200-1210 (2002) and a polynucleotide the sequence of nucleotides of which encodes a dominant non-functional mutated form of the IGF-I receptor. Thus, the dominant non-functional mutated form of the IGF-I receptor solely will be expressed in the cells of the choroid plexus, thus producing the animal model of the present invention. In one particular embodiment, said polynucleotide encodes a dominant non-functional mutated form of the human IGF-I receptor. For illustrative purposes, said dominant non-functional mutated form of the human IGF-I receptor is selected between the non-functional mutated form of the IGF-I receptor referred to as IGF-IR.KR which has the K1003R mutation, in which the lysine residue of the 1003 position of the amino acid sequence of the human IGF-I receptor has been substituted for an arginin residue and the non-functional mutated form of the IGF-I receptor referred to as IGF-IR.KA which has the K1003 mutation, in which the lysine reside of the 1003 position of the amino acid sequence of the human IGF-I receptor has been substituted for an alanin reside (Kato H, Faria TN, Stannard B, Roberts CT, Jr., LeRoith D (1993) Role of tyrosine kinase activity in signal transduction by the insulin-like growth factor-I (IGF-I) receptor. Characterization of kinase-deficient IGF-I receptors and the action of an IGF-I-mimetic antibody (alpha IR-3). J Biol Chem 268: 2655-2661).

Alternatively, said alteration in the biological activity of the IGF-I receptor function in the epithelial cells of the choroid plexus of the cerebral ventricles of said transgenic animals can be produced by the repression of the functional activity of the IGF-I receptor due to the expression of a polynucleotide the sequence of nucleotides of which encodes an element inhibiting the expression of the IGF-I receptor gene capable of repressing the functional activity thereof. As used in the present invention and, as previously stated hereinabove, the term "element inhibiting the expression of the IGF-I receptor gene capable of repressing the functional activity thereof' refers to a protein, enzymatic activity or sequence of nucleotides, RNA or DNA, single or double-strand, which inhibits the translation into protein of the mRNA of the IGF-I receptor. For illustrative purposes, said polynucleotide can be a polynucleotide which encodes a specific sequence of antisense nucleotides of the sequence of the gene or of the mRNA of the IGF-I receptor, or rather a polynucleotide which encodes a specific aptamer of the mRNA of the IGF-I receptor, or rather a polynucleotide which encodes a specific interference RNA ("small interference RNA" or siRNA) of the mRNA of the IGF-I receptor.

Likewise, an animal model of the invention can be produced by conventional transgenesis in which the repression of the functional activity of the IGF-I receptor can be regulated by different mechanisms which would allow for a better control and use of the animal. Thus, one controlled transgenesis technique can consist of the use of the "Cre/Lox" system by means of crossing animals with Lox-IGF-IR (knock-in" systems) transgenic sequences which substitute the endogenous IGF-IR sequence, with animals which have Cre bacterial recombinase controlled by a specific tissue promoter, once again, for example, that of transtyrretin (Isabelle Rubera, Chantal Poujeol, Guillaume Bertin, Lilia Hasseine, Laurent Counillon, Philippe Poujeol and Michel Tauc (2004) Specific Cre/Lox Recombination in the Mouse Proximal Tubule. J Am Soc Nephrol, 15 (8): 2050-6: Ventura A, Meissner A, Dillon CP, McManus M, Sharp PA, Van Parijs L, Jaenisch R, Jacks T. (2004) Cre-lox-regulated conditional RNA interference from transgenes. Proc Natl Acad Sci USA. 101 (28): 10380-5). Another example for generating another controllable transgenic model animal would consist of the use of the "tet-off" system (Rennel E, Gerwins P. (2002) How to make tetracycline-regulated transgene expression go on and off. Anal Biochem. 309 (1): 79-84; Schonig, K. Bujard H. (2003) Generating conditional mouse mutants via tetracycline-controlled gene expression. In: Transgenic Mouse Methods and Protocols, Hofker, M, van Deursen, J (eds.) Humana Press, Totowa, New Jersey, pages 69-104). One embodiment exemplifying the present invention will consist of a Lox-IFT-IR mouse which is crossed with a Tre-Cre mouse - where Tre is the controllable promoter of the Tta protein (tetracycline-controlled transactivator protein): this hybrid subsequently being crossed with a Ttr-Tta mouse such that the resulting mouse: Lox-IGF-IR/Tre-Cre/Ttr-Tta will eliminate the IGF-IR function in response to the administration of tetracycline, a compound which eliminates the action of the Tta protein.

In another aspect, the invention is related to the use of a vector of the invention in a procedure for the production of a non-human animal useful as an experimental model, such as an experimental model of neurodegenerative disease, particularly human neurodegenerative diseases, especially as a model of human neurodegenerative diseases which present with dementia, such as Alzheimer's disease.

The following examples serve to illustrate the invention and must not be considered in a sense of limiting the scope thereof.

### EXAMPLE 1: The creation of a viral vector for sustained transgenic expression.

A viral vector was created as a genetic medium to introduce the mutated IGF-I receptor, referred to as IGF-IR.KR, in epithelial cells in the choroid plexus. IGF-IR.KR, the mutated IGF-I receptor, displays a K1003R mutation, where the lysine residue was substituted for an arginine residue, and acts as a dominant negative in recombination with the normal endogenous receptor, thereby disallowing normal function (Kato H, Faria TN, Stannard B, Roberts CT, Jr., LeRoith D (1993) Role of tyrosine kinase activity in signal transduction by the insulin-like growth factor-I (IGF-I) receptor. Characterization of kinase deficient IGF-I receptors and the main action of an IGF-I mimetic antibody (alpha IR-3), J Biol Chem 268: 2655-2661).

A lentiviral vector with prolonged expression characteristics was used (Consiglio A, Quattrini A, Martino S, Bensadoun JC, Dolcetta D, Trojani A, Bengalia G, Marchesini S, Cestari V, Oliverio A, Bordignon C, Naldini L (2001) In vivo gene therapy of metachromatic luekodystrophy by lentiviral vectors: correction of neuropathy and protection against learning impairments in affected mice. Nat Med 7: 310-316; Kordower JH, Emborg ME, Bloch J, Ma SY, Chu Y, Levanthal L, McBride J, Chen EY, Palfi S, Roitberg BZ, Brown WD, Holden JE, Pyzalski R, Taylor MD, Carvey P, Ling Z, Trono D, Hantraye P, Deglon N, Aebischer P, (2000) Neurodegeneration prevented by lentiviral vector delivery of GDNF in primate models of Parkinson's disease. Science 290: 767-773), derived from the human immunodeficiency type 1 virus (HIV-1), using vesicular stomatitis viruses (VSV) produced by transitory transfection and packaged in 293T cells plasmid vectors, following the concentration of said viral particles using ultra centrifugation, for the viral delivery. A third generation of HIV virus has been created following previously published methods (Dull HB (1998) Behind the AIDS mailer, Am J Prev Med 4: 239-240). For this process four constructions similar to those previously described were employed (Bosch A, Perret E, Desmaris N, Trono D, Heard JM. Reversal of pathology in the entire brain of mucopolysaccharidosis type VII mice after lentivirus-mediated gene transfer. Hum Gen Ther 8: 1139-1150, 2000):
(i) the RSV- Rev non over-lapping vector, which can read the nucleotide sequence which codifies the Rev protein for Roux sarcoma virus (RSV);
(ii) A p-RRE a conditionally cased vector which can read the nucleotide sequence which codifies the Rev response element (RRE);
(iii) A p-VSV vector which can read the nucleotide sequence which codifies the vector's heterogeneous packaging, especially the viral casing for vesicular stomatitis virus (VSV); and
(iv) A transfer vector bearing the genetic construction for the relevant transgene, which in this case is IGF-IR.KR, and the phosphoglycerolkinase (PCK) prompter which permits the transcription vector to be produced efficiently in the packaging cells (293T).
The first three vectors [1)-3)] are known (please refer to the previously quoted references). The construction of the last vector was carried out by introducing a Hincll-Xbal fragment of the IGF-I receptor's cDNA that codifies the IGF-I receptor's mutated form, which in this case is the mutated receptor referred to as IGF-IR.KR which contains the mutation K1003R where the lysine residue has been substituted by an arginine residue (Kato H, Faria TN, Stannard B, Roberts CT, Jr., LeRoith D (1993) Role of tyrosine kinase activity in signal transduction by the insulin-like growth factor-I (IGF-I) receptor. Characterization of kinase deficient IGF-I receptors and the action of an IGF-I mimetic antibody (alpha IR-3) J Biol Chem 268: 2655-2661), in the vector HIV-LacZ ( Naldini L, Blomer U, Gallay P, Ory D, Mulligan R, Gage FH, Verma IM. Trono D (1996) In vivo gene delivery and stable transduction of non-dividing cells by a lentiviral vector. Science 272: 263-267). In concise terms, the cDNA that codifies the mutated form of IGF-I bearing the mutation K1003R (IGF-IR.KR) was introduced into HIV-lacZ via information exchange from lacZ using the cDNA that codifies IGF-IR.KR according to the previously described methodology (Desmaris N, Bosch A, Salaun C, Petit C, Prevost MC, Tordo N, Perrin P, Schwartz O. de Rocquigny H. Heard JM (2001) Production and neurotropism of lentivirus vectors pseudotyped with lyssavirus envelope glycoproteins. Mol Ther 4: 149-156). For this process the HIV-lacZ vector was cut with *Smal*/*Xbal* to eliminate the lacZ cDNA and was then bound with IGF-IR.KR codifying cDNA which was cut with *Hincll*/*Xbal.* The restriction sites are homologous. As a result the transfer vector which bears the transgene IGF-IR.KR was obtained.
The lentiviral vector known as HIV/IGF-IR.KR or HIV/KR in this description was obtained through the transitory transfection of 293T cells. The RSV-Rev, the p-RRE, the p-VSV plasmids and the transfer vector bearing the transgene IGF-IR.KR are episomally packaged in the previously mentioned 293T cells (Desmaris N, Bosch A, Salaun C, Petit C, Prevost MC, Tordo N, Perrin P, Schwartz O. de Rocquigny H, Heard JM (2001) Production and neurotropism of lentivirus vectors pseudotyped with lyssavirus envelope glycoproteins. Mol Ther 4: 149-156). The 293T cellular line (commercially obtainable through the American Type Culture Collection) is a line of transformed epithelial human kidney cells that express the T antigen of SV40, which permits the episomal replication of the plasmids in the prompter region. On previous occasions the cells were planted in 10cm plaques at a density of 1-5 x 10⁶ 24 hours before the transfection in a DMEM environment with 10% of foetal serum and and penicillin (100 IU/ml). During the transfection process a total of 32.75µg of plasmid DNA per plate was used: 3µg of p-VSV plasmids, 3.75µg of RSV-Rev plasmids and 13µg of both p-RRE plasmids and the transfer plasmid bearing the IGF-IR.KR transgene. The precipitate was obtained by adding 500µl of HEPES 2X saline buffer solution (NaCl 280mM, HEPES 100mM, Na₂HPO₄ 1.5mM, pH 7.12) drop by drop. While being shaken the precipitate was added to each cultivation tray. 10 ml of the medium was changed after 24 hours and after a further 24 hours the particles were collected and cleaned using a low speed centrifuge and passed through cellulose acetate filters (0.22µm). Finally, following a series of ultra centrifuge processes the particles or lentiviral vectors HIV/IGF-IR.KR (HIV/KR), were re-suspended in a saline phosphate buffer (PBS/BSA) for later use. In concise terms, firstly the cultivation medium from the trays with the 293T cells was filtered using a 0.45µm filter. This medium was then centrifuged at 4°C for 1.5 hours at 19,000rpm. The precipitate was re-suspended in 1% PBS/PBA and was left for 1 hour in ice and was then re-centrifuged for 1.5 hours at 19,000rpm. The medium was then re-suspended in 1% PBS/BSA and then left in ice for 1 hour and centrifuged at 4°C for 5 minutes at 14,000rpm. The final product was immediately frozen and stored at -80°C. This same method was used to purify the empty HIV particles and the HIV/GFP particles. The empty HIV particles (or the empty HIV vectors), that correspond to the HIV-lacZ cut using *Smal*/*Xbal* and the HIV/GFP particles have been described previously (Desmaris N, Bosch A, Salaun C, Petit C, Prevost MC, Tordo N, Perrin P, Schwartz O, de Rocquigny H, Heard JM (2001) Production and neurotropism of lentivirus vectors pseudotyped with lyssavirus envelope glycoproteins. Mol Ther 4: 149-156).

### EXAMPLE 2: Lentiviral vector expression in epithelial cells from the choroid plexus.

In order to analyse the expression of lentiviral vectors in epithelial cells from the choroid plexus a HIV/GFP lentiviral vector that contained the gene which codifies GFP as a transgene was constructed. In concise terms, the cDNA for the GFP protein gene was sub-cloned in a HIV-1 transfer vector [(pHR'CMV)-PGK in Desmaris N, Bosch A, Salaun C, Petit C, Prevost MC. Tordo N, Perrin P, Schwartz O, de Rocquigny H, Heard JM (2001) Production and neurotropism of lentivirus vectors pseudotyped with lyssavirus envelope glycoproteins. Mol Ther 4: 149-156], in *BsmHI*/*SalI* restriction sites following on from the detailed description from Example 1, where the lentiviral vector referred to as HIV/GFP was obtained.

Following this, the animals, 5-6 month old male rats (n=7), were subjected to an injection using stereotaxical surgery using a Hamilton syringe, under tribromoethanol anaesthetic, containing 6µl of HIV/GFP vector in both side ventricles (stereotaxical coordinates: 1mm from the bregma, 1.2 mm to the side and 4mm deep), at 1µl per minute. Six months later the rats were sacrificed and the presence of the transgene was observed using fluorescence. For this purpose the animal was transcardially perfused with 4% paraformaldehyde. Then the brain was vibratome cut in 50µm sections, and the sections were immediately mounted on gelatinized holders and the fluorescence of the GFP protein was directly observed using a fluorescence microscope (Leica).

As a result it was determined that by administering the HIV/GFP lentiviral vector (the vector used in the invention of the codifying gene for the fluorescent GFP protein used as a transgene) to adult rats via inlracerebroventricular (icv) injections results in the sustained expression of the GFP protein in the choroid plexus (Figure1).

### EXAMPLE 3: The transformation of epithelial cells from the choroid plexus using the lentiviral vector HIV/IGF-IR,KR (HIV/KR)

The single layer of epithelial cells was obtained using a previously described method (Strazielle, N. and Ghersi-Egea, J.F. (1999) Demonstration of a coupled metabolism-efflux process at the choroid plexus as a mechanism of brain protection toward xenobiotics. J. Neurosci. 19: 6275-6289). 5-7 day old rats were sacrificed and the choroid plexus from the side and fourth ventricles were rapidly extracted and set in a DMEM cultivation medium on ice. Following their extraction and preparation the plexuses were digested using enzymes; 1 mg/ml of pronase (SIGMA) and 12,5µg/ml Dnase I (Boehringer Mannheim), using simultaneous mechanical dispersion over a 15 minute period. Finally the solution was centrifuged (1,000rpm) and the cells were re-suspended in DMEM with a 10% foetal serum (FCS) supplement, 10ng/ml of EGF (Epidermal Growth Factor) (Sigma), 5ng/ml of FGF (Fibroblast Growth Factor) (Boehringer Mannheim) and gentamicin, These cells were transformed with the lentiviral vector HIV/IGF-IR.KV (HIV/KR) and the empty HIV vector, using the following summarised method. After 24 hours of cultivation the medium was changed with fresh DMEM containing the virus (at least 50µg/ml diluted at between 10⁻² and 10⁻³) and 8µg/ml of polybrene (Sigma). This infective medium was replaced after 24 hours and the cells were maintained for another day and finally following suction of the medium the cells were processed.

As a result the addition of the lentiviral vector HIV/IGF-IR:K (HIV/KR) to epithelial cells in cultivations obtained from rat choroid plexus was observed to produced a lower rate of the trophic factor IGF-1. Only in the cells infected with the HIV/KR vector, not those transfected with the empty HIV vector did the IGF-I fail to produce peptide transcytosis Aβ1-40 (Figure 2). The transcytosis was quantified according to the amount of Aβ1-40 which passed from the upper cultivation chamber to the lower cultivation chamber, required the crossing of a single layer of epithelial cells (Carro E, Trejo JL, Gomez-Isla T, LeRoith D, Torres-Aleman I (2002) Serum insulin-like growth factor I regulates brain amyloid-beta levels. Nat Med 8: 1390-1937).

### EXAMPLE 4: The development of an Alzheimer type neuropathology in healthy adult rats

Healthy adult rats were infected with Wistar strain using the HIV/IGF-IR.KR (HIV/KR) vector. This process was carried out using stereotaxical Surgery with a Hamilton syringe under tribromoethanol anaesthetic, containing 6µl of HIV/IGF-IR.KR vector in both side ventricles (stereotaxical coordinates: 1 mm from the bregma, 1.2 mm to the side and 4mm deep), at 1µl per minute on 5-6 month old male rats. The control animals were injected with the same quantity of empty HIV viral vector under the same conditions. 5 months later the rats' cognitive capacity was measured using the Morris spatial learning test which relies on the hippocampus, a structure typically affected in Alzheimer (Clark CM, Karlawish JH (203) Alzheimer disease: current concepts and emerging diagnostic and therapeutic strategies. Ann Intern Med 138: 400-410), following standardized methodology (Trejo JL, Torres Aleman I (2001) Circulating insulin-like growth factor I mediates exercise-induced increases in the number of new neurons in the adult hippocampus. J Nuerosci 21: 1628-1634). This test known as the "water maze" (or the Morris test) determines spatial memory (van der Staay FJ (2002) Assessment of age associated cognitive deficits in rats: a tricky business. Nuerosci Biobehav Rev 26: 753-759), which is one of the characteristic deficits presented in Alzheimer disease. On completion of the test the rats were sacrificed (6 months after being injected with the viral vector) and perfused via the aorta artery with saline buffer and their brains were immediately extracted, one hemisphere was stored at -80°C for later processing using "western blot" and the other hemisphere was immersed in 4% paraformaldyhde for 24 hours for an immunohistochemistry study.

The levels of cerebral amyloid (Aβ) and the levels of LCR liquid were determined using western blot techniques, ELISA and using immunocytochemistry, following previously described methodology ( Carro E, Trejo JL, Gomez-Isla T, LeRoith D, Torres-Aleman I (2002) Serum insulin-like growth factor I regulates brain amyloid-beta levels. Nat Med 8: 1390-1937) and the levels of tau hyperphosphorylate (HPF-tau) in the cortex were also measured using western blot and immunocytochemistry (Carro E, Trejo JL, Gomez-Isla T, LeRoith D, Torres-Aleman 1 (2002) Serum insulin-like growth factor I regulates brain amyloid-beta levels. Nat Med 8: 1390-1937). In addition, the presence of HPF-tau deposits and amyloid deposits was also recorded using immunocytochemistry techniques (Carro E, Trejo JL, Gomez-Isla T, LeRoith D, Torres-Aleman I (2002) Serum insulin-like growth factor I regulates brain amyloid-beta levels. Nat Med 8: 1390-1937).

The animals with the blocked IGF-I signal within the choroid plexus due to the addition of the HIV/IGF-IR.KR vector showed significant cognitive deficits in spatial learning and memory (Figures 3A and 3B).

In addition a significant increase in the levels of Aβ was observed within the cerebral parachemistry compared to control animals and at the same time lower Aβ levels were observed in the LCR (Figures 4A and 4B). Both alterations are typical in Alzheimers disease (Selkoe DJ (2001) Clearing the Brain's Amyloid Cobwebs. Neuron 32: 177-180; Sunderland T, Linker G, Mirza N, Putnam KT, Friedman DL, Klmmel LH, Bergeson J, Manetti GJ, Zimmermann M, Tang B, Bartko JJ, Cohen RM (2003) Decreased beta-amyloid1-42 and increased tau levels in cerebrospinal fluid of patients with Alzheimer's disease. JAMA 289: 2094-2103). Along with this amyloidosis an intra and extra cellular HPF-tau accumulation was observed in telencephalic regions (Figure 5). The extra cellular accumulations also contain ubiquitin (Figure 5C) and are also characteristic in Alzheimer disease (Clark CM, Karlawish JH (2003) Alzheimer disease: current concepts and emerging diagnostic and therapeutic strategies. Ann Intern Med 138: 400-410). In addition, the animals showed Alzheimer type cellular alterations as they were seen to present reactive gliosis in association with the protein deposits and the significant synaptic protein deficits (Masliah E, Mallory M, Alford M, DeTeresa R, Hansen LA, McKeel DW, Jr., Morris JC (2001) Altered expression of synaptic proteins occurs early in the progression of Alzheimer disease. Neurology 56: 127-129). In conclusion, the animals injected with the prolonged expression lentiviral vector HIV/IGF-IR.KR (HIV/KR) presented neuropathological characteristics associated with Alzheimer's disease such as: high cerebral levels of amyloid, the presence of intra and extra cellular deposits of tau hyperphosphorylate and ubiquitin and cognitive deficiency.

### EXAMPLE 5: The development of the Alzheimer type neuropathology In genetically modified mice LID mice)

Another example of the experiment consisted in producing Alzheimer type pathological changes in transgenic mice. The chosen mice were old mice, to better simulate the normal conditions in which the Alzheimer pathology is developed in human beings.

The HIV/IGF-IR.KR (HIV/KR) vector was injected in 15 month old or older LID genetically modified transgenic mice. The transgenic mice used in this example are deficient in seric IGF-I following the elimination of the IGF-I hepatic gene using the Cre/Lox system (LID mice) (Yakar S, Liu JL, Stannard B, Butler A, Accili D, Sauer B, LeRoith D (1999) Normal growth and development in the absence of hepatic insulin-like growth factor I. Proc Natl Acad Sci U S A 96: 7324-7329). LID mice already show some characteristics of Alzheimers per se, as the IGF-I deficit generates amyloidosis and gliosis (Carro E, Trejo JL, Gomez-Isla T, LeRoith D, Torres-Aleman I (2002) Serum insulin-like growth factor I regulates brain amyloid-beta levels. Nat Med 8: 1390-1937). In addition as the mice were old they showed cognitive deficiency and amyloidosis (Bronson RT, Lipman RD, Harrison DE (1993) Age-related gliosis in the white matter of mice. Brain Res 609: 124-128; van der Staay FJ (2002) Assessment of age associated cognitive deficits in rats: a tricky business. Nuerosci Biobehav Rev 26: 753-759). The objective of this experiment was to obtain the most favourable conditions for amyloidosis production to determine if the system provided for this experiment generates amyloid deposits, one of the characteristics of Alzheimer's disease. The procedure and reactive material used are described in the previous examples. The animals were sacrificed three months after being injected with the viral vector.

Just three months after the administration of the HIV/KR vector the old LID mice showed severe cognitive deficiency (Figure 6A), and amyloidosis and taupathy similar to that observed in adult rats six months after being exposed to the viral vector (the results are similar to those described in Figures 4 and 5 although the data is not included). More importantly using this model a much more advanced state of the disease is achieved: the animals show amyloid accumulations, which although not congophilic (they are not detected with the insoluble plaque marker "Congo red") they display typical diffused plaques (Figure 6B).

## Claims

1. A non-human animal, useful as an experimental model is **characterised by** the display of an alteration in the biological activity of the receptor of the insulin type I-like growth factor (IGF-I) located in the epithelial cells in the choroids plexus from the cerebral ventricles.

2. An animal which according to claim 1, is **characterized** due to said alteration of the biological activity of the IGF-I receptor consisting in biological elimination.

3. An animal according to both claim 1 and claim 2, **characterised** as a mammal.

4. An animal according to claim 3 **characterised** due to selection between rodents and primates.

5. An animal according to claim 4, **characterised by** being a rat or a mouse

6. An animal according to claims 1-5, **characterised** due to said alteration in the IGF-I receptor functions in the epithelial cells located in the choroids plexus which is due to the expression of a dominant non functional mutated form of said IGF-I receptor.

7. An animal according to claim 6. **characterised by** the fact that the afore mentioned dominant non functional mutated form of the IGF-I receptor is the non functional mutated form of the IGF-I receptor referred to as IGF-IR.KR which displays the K1003R mutation, in which the lysine residue found in position 1003 in the IGF-I receptor amino acid sequence has been substituted by an arginine residue.

8. An animal which according to claim 6, is **characterised** as said dominant non functional mutated form of the IGF-I receptor is the mutated form of the IGF-I non functional receptor referred to as IGF-IR,KR which contains the K1003A mutation, in which the lysine residue in position 1003 of the receptor amino acids sequence for the human IGF-I has been substituted with an alanine residue.

9. An animal according to any one of the claims from 1-8, is **characterised** as a normal animal.

10. An animal which according to claim 9 is **characterised** as a normal healthy rat.

11. An animal which according to claim 11 is **characterised** due to the fact that said animal is transgenic.

12. An animal which according to claim 11 is **characterised** due to the fact that said transgenic animal is an LID transgenic mouse.

13. An animal which according to anyone of the claims from 1-12, is useful as an experimental model for a neurodegenerative disease.

14. An animal which according to claim 13, where said neurodegenerative disease is Alzheimer's disease.

15. A procedure for the obtaining of a non human animal useful as an experimental model according to any of the claims 1-14, that includes the elimination of the functional activity of the IGF-I receptor in epithelial cells in the choroid plexus in said non human animal using a transgenesis process.

16. A procedure which according to claim 15, for said transgenesis functional activity elimination process for the IGF-I receptor includes the administration of epithelial cells from the choroids plexus of a non human animal developed with a genetic make-up that includes a polynucleotide with a nucleotide sequence that codifies a dominant non functional mutated form of the IGF-I receptor, or a vector that can read said genetic structure to enable the transformation of said epithelial cells from the choroid plexus in a way which expresses said dominant non functional mutated form of the IGF-I receptor.

17. A procedure which according to claim 16, in which the administration of said genetic construction or said vector to said epithelial cells from the choroid plexus will be carried out using a intracerebroventricular injection (icv).

18. A procedure which according to claim 16, in which said vector is selected from viral and non viral vectors.

19. A procedure which according to claim 18, which the viral vector is a lentiviral vector.

20. A procedure which according to claim 16, where said dominant non functional mutated form of the IGF-I receptor is the mutated form of the IGF-I non functional receptor referred to as IGF-IR.KR which contains the K1003R mutation, in which the lysine residue in position 1003 of the IGF-I receptor amino acids sequence has been substituted with an arginine residue.

21. A procedure which according to claim 16, where said dominant non functional mutated form of the IGF-I receptor is the mutated form of the IGF-I non functional receptor referred to as IGF-IR.KR which contains the K1003A mutation, in which the lysine residue in position 1003 of the human IGF-I receptor amino acids sequence has been substituted with an alanine residue.

22. A procedure according to claim 16, in which said animal is a normal non-human animal,

23. A procedure which according to claim 16, in which said non human animal is a non human transgenic animal.

24. A procedure which according to claim 15, in which said transgenesis process for the elimination of the functional activity of the IGF-I receptor includes the transformation of the epithelial cells from the choroids plexus of a non-human animal by introducing a genetic construction which can interpret a polynucleotide whose nucleotide sequence codifies an inhibition element on the expression of IGF-I receptor gene capable of eliminating it's biological activity, or a vector which includes said genetic construction, where the inhibitor element is selected from:
a) a sequence of antisense nucleotides specifies the gene sequence or the sequence for the IGF-I mRNA receptor,
b) A specific mRNA ribozyme from the IGF-I receptor,
c) A specific mRNA aptamer from the IGF-I receptor and,
d) A specific mRNA RNA interference (RNAi) from the IGF-I receptor

25. A lentiviral vector obtained by transitory transfection in package cells with:
A plasmid (i) which can read the sequence of nucleotides selected from:
- a sequence of nucleotides that codify the dominant non functional mutated form of the IGF-I receptor, and
- a sequence of nucleotides that codify an inhibitor element for IGF-I receptor gene expression capable of eliminating functional activity:
A plasmid (ii) that includes the sequence of nucleotides which codify the Rev protein;
A plasmid (iii) that includes the sequence of nucleotides that codify the Rev response element (RRE); and
A plasmid (iv) that includes the sequence of nucleotides that codify the heterogeneous vector casing.

26. The vector according to claim 25, in which said plasmid (i) is a plasmid that can read the sequence of nucleotides that codify the non functional mutated form of the IGF-I receptor selected form a sequence of nucleotides that codify the non functional mutated for of the IGF-I receptor referred to as IGF-IR.KR which presents the mutation K1003R, where the lysine residue in position 1003 of the sequence of amino acids for the IGF-I human receptor has been substituted for arginine residues and the nucleotide sequence that codifies the non functional mutated form of the IGF-I receptor referred to as IGF-IR.KR showing the K1003A mutation, in which the lysine residue in position 1003 of the amino acid sequence for the human IGF-I receptor has been substituted for an alanine residue.

27. The vector according to claim 25 in which the plasmid (ii) is a is a plasmid that can read the sequence of nucleotides that codify an inhibitor element for IGF-I receptor gene expression capable of eliminating functional activity between a sequences of nucleotides that codify: a) an antisense nucleotide sequence specific to the gene sequence or to the IGF-I receptor mRNA, b) a ribozyme specific to the IGF-I receptor mRNA, c) a specific aptamer for the IGF-I receptor mRNA and d) RNA interference (RNAi) specific to the IGF-I receptor mRNA.

28. The viral vector according to claim 25, in which: said package cells are 293T cells; said plasmid (i) is the plasmid defined in claim 27 or 28; said plasmid (ii) is the plasmid identified as RSV-Rev, that can read the sequence of nucleotides that codify the Rev protein for the Rous Sarcoma virus (RSV); said plasmid (iii) is the plasmid identified as p-RRE, that can read the nucleotide sequence that codifies the casing protein for the vesicular stomatitis virus (VSV).

29. The use of the vector according to any of the claims from 25-28, in procedures for obtaining the non human animal, useful as an experimental model, according to any of the claims from 15-24.

30. The procedure according to claim 15, in the transgenesis process for the elimination of IGF-I functional activity in epithelial cells from the choroid plexus includes the administration of a genetic construction able to read the specific prompter for the choroid plexus and a polynucleotide whose sequence codifies the dominant non functional mutated form of the IGF-I receptor, or a vector that can read said genetic construction, from embryonic cells from the non human animal.

31. A procedure where according to claim 30, said dominant non functional mutated form of the IGF-I receptor is the mutated form of the IGF-I non functional receptor referred to as IGF-IR.KR which contains the K1003R mutation, in which the lysine residue in position 1003 of the IGF-I receptor amino acids sequence has been substituted with an arginine residue.

32. A procedure where according to claim 30, where said dominant non functional mutated form of the IGF-I receptor is the mutated form of the IGF-I non functional receptor referred to as IGF-IR.KR which contains the K1003A mutation, in which the lysine residue in position 1003 of the human IGF-I receptor amino acids sequence has been substituted with an alanine residue.

33. The procedure according to claim 15, where the said transgenesis elimination process for IGF-I functional activity in epithelial cells from the choroid plexus includes the administration of a genetic construction able to read the specific prompter for the choroid plexus and a polynucleotide whose sequence codifies the dominant non functional mutated form of the IGF-I receptor, or a vector that can read said genetic construction, from embryonic cells from the non human animal, where the inhibitor element is selected from:
a) a sequence of antisense nucleotides specifies the gene sequence or the sequence for the IGF-I mRNA receptor,
b) A specific mRNA ribozyme from the IGF-I receptor,
c) A specific mRNA aptamer from the IGF-I receptor and,
d) A specific mRNA RNA interference (RNAi) from the IGF-I receptor

34. Procedure according to claim 30-33, where said prompter specific to the tissue is a transthyretin gene prompter.

35. Procedure according to claim 30-34, in which said transgenesis process is non-deductible.

36. The use of the animal according to any of the claims from 1-14 as a model for the study of ethiopathogenic mechanisms in neurodegenerative diseases or for the identification and evaluation of therapeutic compounds for said disease.

37. The use according to claim 36, in which said neurodegenerative disease is a neurodegenerative disease leading to dementia.

38. The use according to claim 36 or 37, in which said neurodegenerative disease is a human neurodegenerative disease.

39. The use according to claim 36, of an animal according to any of the claims from 1-14 as a model for the study of ethiopathogenic mechanisms in Alzheimer's disease or for the identification and evaluation of any therapeutic compounds for combating said disease.
